# EUROPEAN PATENT APPLICATION

(11) **EP 1 284 147 A2**
(43) Date of publication of application: **19.02.2003**
(21) Application number: 02015975.2
(22) Date of filing: 18.07.2002
(51) Int. Cl.: A61N 1/05

(54) **Preformed three-pole/four-pole lead for cardiac stimulation**

(30) Priority: 06.08.2001 IT PD20010198
(71) Applicant: Medico S.p.a., 35121 Padova (IT)
(72) Inventor: Snichelotto, Eugenio, 35121 Padova (IT); Bardaldo, Marco, 35121 Padova (IT); Di Gregorio, Franco, 35121 Padova (IT)
(74) Representative: Gustorf, Gerhard, Dipl.-Ing.

(57) **Abstract**

The invention is a new implantable lead for cardiac stimulation with single-pole and/or two-pole connectors at one end, having a wholly or partly spiral shape, with at least two curves on two distinct planes and three or more electrodes, of which two situated at the tip and two, close together, situated along the lead.

## Description

### FIELD OF THE INVENTION

This patent concerns medical devices and in particular it concerns a new preformed lead for intracardiac stimulation.

Permanent stimulation of some cardiac muscles is necessary in certain cardiac pathologies.

### PRIOR ART

Among the possibilities of permanent heart stimulation, one that is particularly convenient is the solution that contemplates efficacious stimulation of the atrium and of the ventricle, normally possible by implanting two leads.

The usual technique contemplates the use of two separate leads, single-pole or two-pole, inserted through the veins leading to the heart until the respective electrodes are positioned in suitable points.

In particular, one or two electrodes are placed in contact with the tissue of the atrial wall, while a third or fourth electrode is placed in contact with the tissue of the ventricular wall.

This solution is not completely free of drawbacks.

It is necessary to implant two leads which cannot always be introduced through a single vein, so that they have to be inserted through two separate veins. This double insertion constitutes a greater shock for the patient's circulatory system.

Moreover, the implanting of the two leads involves a greater complexity of the surgical operation, with consequent less reliability.

The two electrodes for the atrial wall require constant contact with said wall. The traditional three-pole/four-pole single leads used have a linear form and are flexible but, for obvious reasons, they do not adhere constantly to the atrial walls (floating electrodes). Even if these electrodes come away from the wall, they detect spontaneous atrial activity, but they do not allow reliable electric stimulation of the atrium.

So, at the present date, the sequential stimulation of atrium and ventricle (DDD pacing) requires the use of two different leads, one for the atrium and one for the ventricle, both provided with electrodes at the tip.

### DESCRIPTION

To overcome the above mentioned drawbacks, a new type of preformed three-pole/four-pole single lead for cardiac stimulation has been studied and implemented.

The aim of the new preformed three-pole/four-pole lead is to guarantee the correct and permanent contact of all the electrodes with the required points on the atrial and ventricular walls.

Another purpose of the new preformed lead is to combine all three electrodes in a single lead, in order to insert only one lead in the veins leading to the heart and to limit the shock to the patient's circulatory system.

Implantable lead for cardiac stimulation equipped with three/four electrodes, of which one or two at the tip and two at a suitable distance from the tip and from each other, having a spiral shape or presenting at least two curves on two distinct planes.

The characteristics of the preformed lead for cardiac stimulation will be more clearly shown in the following description with reference to the drawings enclosed as an example without limitation.

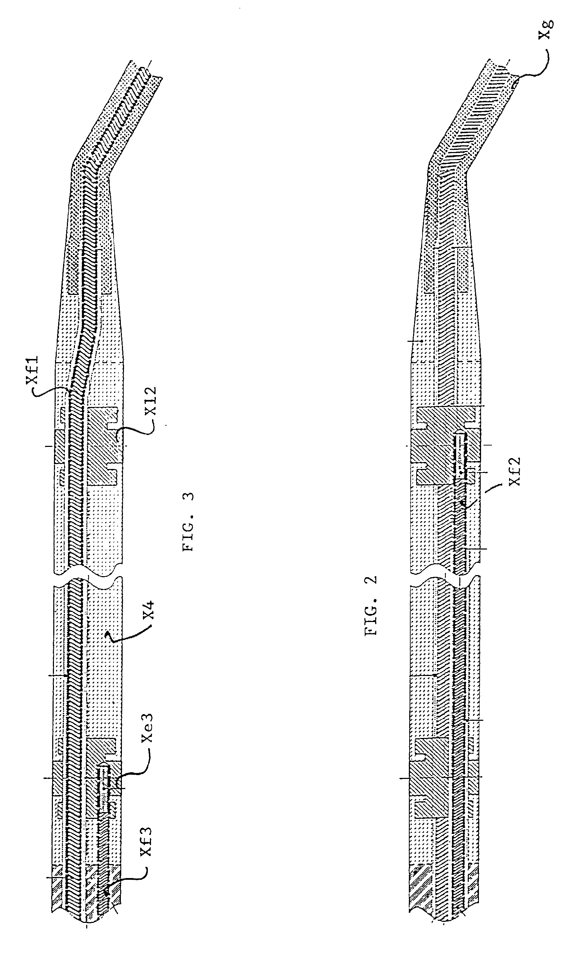

Figure 1 and figure 1a show a side view of the new lead (X), while figures 2 and 3 show two partial sections of the same.

Figures 4 and 5 illustrate two possible configurations of the new lead (X) and its respective applications in the atrium and in the ventricle of the heart (C).

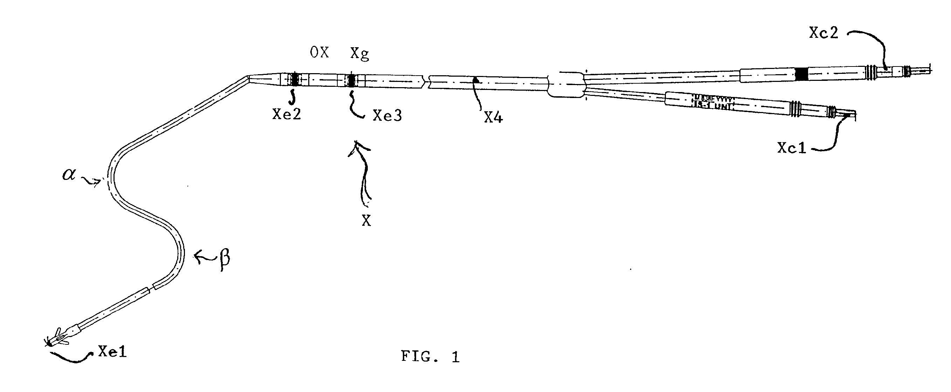

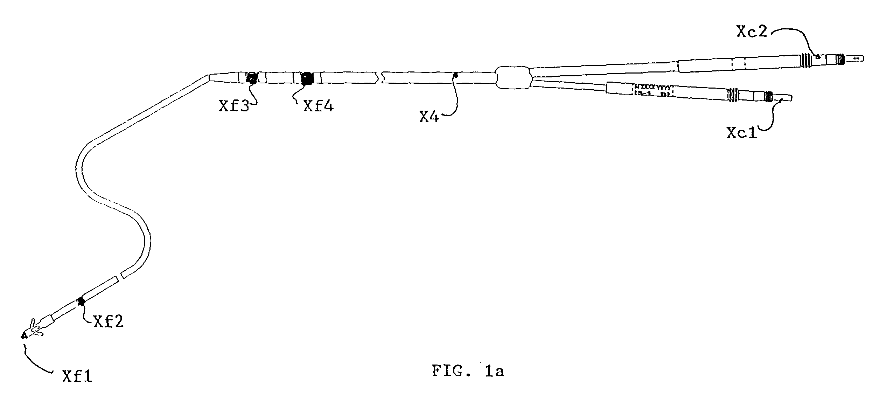

The new lead (X) is composed of a linear sheath (Xg) having electrodes (Xe) at the ends and/or along the sheath (Xg). Figure 1 shows three electrodes (Xe) of which one (Xe1) at one end and the other two (Xe2, Xe3) along the sheath (Xg) at a suitable distance from the tip electrode (Xe1) and properly distanced from each other; figure 1a shows 4 electrodes, two of which (xf1, xf2) at one end and the other two (Xf3, Xf4) along the sheath (Xg).

The electrode (Xe1) at the end, subsequently referred to as the tip electrode, is intended to adhere to the ventricular wall, while the other two electrodes (Xe2, Xe3), subsequently referred to as the atrial electrodes, are intended to adhere to the atrial wall.

At the opposite end are two connectors (Xc1, Xc2), one single-pole (Xc1) and one two-pole (Xc2), for the three electrodes (Xe1, Xe2, Xe3).

Inside the sheath (Xg) are incorporated the electric wires (Xf1, Xf2, Xf3) for connecting the electrodes (Xe1, Xe2, Xe3) to the respective connectors (Xc1, Xc2).

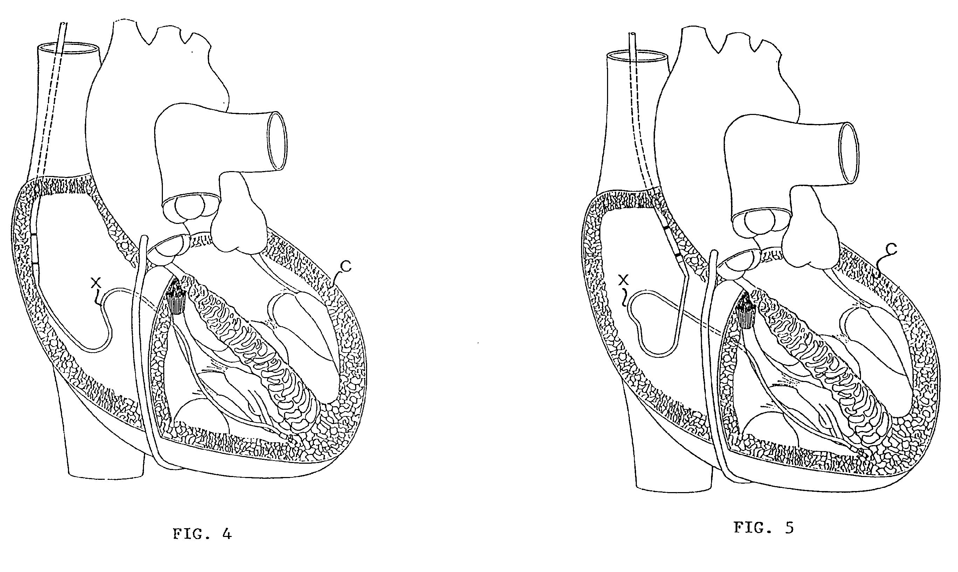

The sheath (Xg) and the entire lead (X) have a multi-arched conformation such that, once the new lead (X) is inserted in the heart (C), the tip electrode (Xe1) and the atrium electrodes (Xe2, Xe3) are in the required positions (figures 4, 5). In particular the new lead (X) is, partly or completely, spiral-shaped and in any case it has at least two curves (α) (β) on two distinct planes.

Moreover, said shape of the new lead (X), is such that it rests on various points in the heart (C), so that any movement of the heart (C) and external stress do not modify the spatial position of the lead (X) itself and of the electrodes (Xe1, Xe2, Xe3).

The various parts of the new lead (X) are made of suitable materials: the sheath (Xg) is made preferably of moulded rubber and/or silicone tube, while the electric leads (Xf1, Xf2, Xf3) are made of metal spiral.

These materials allow a general forming of the new lead (X) during production, as well as the adaptation of its shape to specific needs.

Therefore, with reference to the above description and to the enclosed drawings, the following claims are expressed.

## Claims

1. Implantable lead for cardiac stimulation with single-pole and/or two-pole connectors (Xc1, Xc2) at one end, **characterised in that** it has a spiral shape, wholly or partly.

2. Implantable lead for cardiac stimulation with single-pole and/or two-pole connectors at one end, **characterised in that** it has at least two curves (α) (β) on two distinct planes.

3. Lead according to claim 1 or 2, **characterised in that** it is provided with three or more electrodes, of which two (Xf1, Xf2) situated at the tip and two (Xf3, Xf4), close together, situated along the lead.
